# EUROPÄISCHE PATENTANMELDUNG

(11) **EP 1 898 106 A1**
(43) Veröffentlichungstag der Anmeldung: **12.03.2008**
(21) Anmeldenummer: 07016512.1
(22) Anmeldetag: 23.08.2007
(51) Int. Cl.: F16B 7/02, A61M 39/10, F16B 7/00, A61M 25/00

(54) **Adapter für eine Harnsammelvorrichtung**

(30) Priorität: 06.09.2006 DE 202006013768 U
(71) Anmelder: Scheu, Rolf Rainer, 60488 Frankfurt/Main (DE); Sabeff, Christian, 1210 Wien (AT); Milacek, Walter, 1020 Wien (AT)
(72) Erfinder: Scheu, Rolf Rainer, 60488 Frankfurt (DE)
(74) Vertreter: Müller-Gerbes, Margot

(57) **Zusammenfassung**

Die Erfindung betrifft einen Adapter für eine Harnsammelvorrichtung, die einen Blasenkatheter (1) mit proximalem (10) und distalem (11) Ende sowie ein Sammelbehältnis (4) mit Verbindungsschlauch (40) umfasst, wobei der Verbindungsschlauch über einen Stufenkonnektor (3) an das proximale Ende des Blasenkatheters unter Ausbildung eines vom distalen Ende bis zum Sammelbehältnis durchgehenden Lumens (L) anschließbar ist, wobei der Adapter unter Verlängerung des Lumens zwischen dem proximalen Ende des Blasenkatheters und dem Stufenkonnektor oder dem Stufenkonnektor und dem Verbindungsschlauch einsetzbar ist und einen in das Lumen einmündenden Messkanal für eine Druckmessung aufweist.

## Beschreibung

Die Erfindung betrifft einen Adapter für eine Harnsammelvorrichtung, die einen Blasenkatheter mit proximalem und distalem Ende sowie ein Sammelbehältnis mit Verbindungsschlauch umfasst, wobei der Verbindungsschlauch über einen Stufenkonnektor an das proximale Ende des Blasenkatheters unter Ausbildung eines vom distalen Ende bis zum Sammelbehältnis durchgehenden Lumens anschließbar ist.

Harnsammelvorrichtungen der eingangs genannten Art sind in der klinischen Praxis bekannt und werden beispielsweise dazu eingesetzt, sogenannte intra-abdominale Druckmessungen (IAP) durchzuführen, um z.B. bei Entzündungen eine Druckmessung zum Körperinnern durchzuführen. Zu diesem Zweck werden bislang speziell für die IAP-Messung ausgestaltete Stufenadapter innerhalb der Harnsammelvorrichtung verwendet, die über einen integrierten Anschluss einer Druckmessleitung für die Gewinnung des IAP-Messwertes verfügen. Demzufolge ist es im Falle einer gewünschten IAP-Messung erforderlich, an den Blasenkatheter ein neues Sammelbehältnis anzuschließen, dessen Verbindungsschlauch mit einem solchen speziellen Stufenkonnektor ausgerüstet ist. Dies ist umständlich und kostenintensiv.

Aufgabe der vorliegenden Erfindung ist es daher, einen Adapter für eine Harnsammelvorrichtung der eingangs genannten Art vorzuschlagen, mit welchem die geschilderten Probleme und Nachteile umgangen werden.

Zur Lösung der gestellten Aufgabe wird erfindungsgemäß vorgeschlagen, dass der Adapter unter Verlängerung des Lumens zwischen dem proximalen Ende des Blasenkatheters und dem Stufenkonnektor oder dem Stufenkonnektor und dem Verbindungsschlauch einsetzbar ist und einen in das Lumen einmündenden Messkanal für eine Druckmessung, vorzugsweise eine intra-abdominale Druckmessung aufweist.

Der erfindungsgemäße Adapter ermöglicht es somit, eine konventionelle Harnsammelvorrichtung, bestehend aus Blasenkatheter, Sammelbehältnis mit Verbindungsschlauch sowie einem diese Teile verbindenden Stufenkonnektor bei Bedarf durch Zwischenfügung des erfindungsgemäßen Adapters dahingehend zu modifizieren, dass eine Druckmessleitung anschließbar und von daher eine Druckmessung möglich wird, ohne dass hierfür eine spezielle Harnsammelvorrichtung herangezogen werden muss. Vielmehr wird bei bereits gelegtem Blasenkatheter einfach bei Bedarf der erfindungsgemäße Adapter an der gewünschten Stelle zwischengeschaltet, wodurch die Druckmessung ermöglicht wird, gleichzeitig aber die weitere Ableitung von Harn aus dem Blasenkatheter in das Sammelbehältnis aufrechterhalten bleibt.

Somit wird für den Fall einer gewünschten Druckmessung lediglich ein erfindungsgemäßer Adapter benötigt, da derartige Patienten meist ohnehin bereits über einen gelegten Blasenkatheter mit herkömmlichem Sammelbehältnis verfügen.

Hierdurch werden der Arbeitsaufwand und die Kosten einer derartigen Untersuchung ernorm verringert und für den Patienten eine erhebliche Verringerung seiner Belastung erreicht.

Vorteilhafte Ausgestaltungen und Weiterbildungen der Erfindung sind Gegenstand der abhängigen Ansprüche.

Gemäß einer Ausführungsform des erfindungsgemäßen Adapters ist er für den Einsatz zwischen Blasenkatheter und Stufenkonnektor vorgesehen und weist auf seiner dem proximalen Ende des Blasenkatheters zugewandten Seite ein abgestuftes Außenprofil zum Aufstecken des proximalen Endes des Blasenkatheters und auf seiner dem Stufenkonnektor zugewandten Seite eine sich konisch verengende Einstecköffnung zum Einstecken des Stufenkonnektors auf.

Alternativ kann der erfindungsgemäße Adapter auch für den Einsatz zwischen dem Stufenkonnektor und dem Verbindungsschlauch vorgesehen sein und weist dann auf seiner dem Stufenkonnektor zugewandten Seite vorteilhaft einen zylindrischen Einsteckvorsprung zum Einstecken in den Stufenkonnektor und auf seiner dem Verbindungsschlauch zugewandten Seite eine Aufnahmebohrung zum Einsetzen des freien Endes eines Verbindungsschlauches auf.

In jedem Falle lässt sich der erfindungsgemäße Adapter an einem konventionellen Stufenkonnektor, Blasenkatheter und Verbindungsschlauch und Sammelbehältnis verwenden, ohne dass auf spezielle Ausgestaltungen in Verbindung mit der gewünschten Druckmessung zurückgegriffen werden müsste.

In einer bevorzugten Ausgestaltung der Erfindung ist der Adapter ausgehend vom Messkanal auf seiner dem proximalen Ende des Blasenkatheters abgewandten Seite mit einer Umfangsnut ausgebildet. In dieser vorzugsweise unmittelbar benachbart zur Messleitung ausgebildeten Umfangsnut kann eine Abklemmvorrichtung, z.B. eine Schiebeklemme an einer durch die Umfangsnut eindeutig definierten Stelle positioniert, insbesondere vormontiert werden.

Die Schiebeklemme, welche bei Schlauchsystemen zum Freigeben bzw. Verschließen des Schlauchquerschnittes allgemein bekannt ist, ermöglicht in ihrer geöffneten Position die ungehinderte Ableitung von Harn aus dem Blasenkatheter in das Sammelbehältnis.

Wird eine Druckmessung durchgeführt, so wird zunächst die Schiebeklemme verschlossen, um eine weitere Harnableitung in das Sammelbehältnis zu unterbinden. Hierbei gestattet die Umfangsnut durch die Querschnittsverringerung des Adapters zum einen den zuverlässigen Verschluss des Querschnitts, zum anderen legt sie die Position des mit der Schiebeklemme bewirkten Verschlusses eindeutig und wiederholgenau fest, so dass zum Zwecke der Druckmessung stets ein definiertes Volumen im Blasenkatheter, Adapterteilabschnitt und der Messleitung zur Verfügung steht. Wäre die Position der Schiebeklemme nicht durch eine Umfangsnut festgelegt, könnte die Position des Querschnittsverschlusses variieren und damit auch das für die Druckmessung genutzte Volumen, so dass sich Messfehler einstellen.

Nach dem erfolgten Verschluss der Verbindung zum Sammelbehältnis wird das verbleibende System aus Messleitung, Adapterteilabschnitt, Blasenkatheter und der Blase des Patienten über die Messleitung mit einem definierten Volumen an Flüssigkeit, z.B. NaCl gefüllt und anschließend die eigentliche Druckmessung durchgeführt.

Nach Abschluss der Messungen kann die Schlauchklemme wieder geöffnet werden und eine Ableitung von Harn in das Sammelbehältnis erfolgen.

Der erfindungsgemäße Adapter kann bevorzugt aus einem geeigneten thermoplastischen oder elastomeren Kunststoff beispielsweise als Spritzgussteil gefertigt werden, so dass er in hohen Stückzahlen wirtschaftlich herstellbar ist. Beispiel für einen geeigneten Kunststoff ist ein Silikon oder ein Elastomer mit einer Shore-Härte A von 40 bis 90.

Die Erfindung umfasst des Weiteren auch eine Harnsammelvorrichtung der vorangehend erläuterten Art, die mit einem erfindungsgemäßen Adapter ausgestattet ist.

Weitere Ausgestaltungen und Einzelheiten der Erfindung werden nachfolgend anhand der Ausführungsbeispiele darstellenden Zeichnung näher erläutert. Es zeigen:
- Figur 1: in schematisierter Übersichtsdarstellung eine Harnsammelvorrichtung,
- Figur 2: die Einzelheit X der Harnsammelvorrichtung gemäß Figur 1 in einer ersten Ausführungsform,
- Figur 3: die Einzelheit X der Harnsammelvorrichtung gemäß Figur 1 in einer zweiten Ausführungsform,
- Figur 4: eine weitere Ausführungsform der Erfindung.

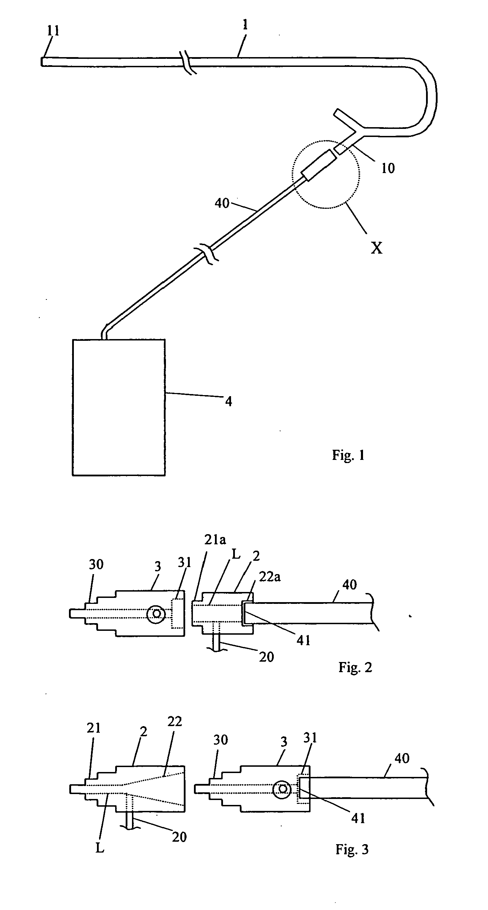

In der Figur 1 ist eine Harnsammelvorrichtung dargestellt, die in an sich bekannter Weise einen Blasenkatheter 1 mit einem distalen Ende 11 zum Einführen in einen Patienten und einem proximalen Ende 10 umfasst, welches außerhalb des Patienten zum Liegen kommt und im dargestellten Ausführungsbeispiel als Y-Endstück ausgeführt ist, dessen einer Ast mit einem nicht dargestellten Stopfen verschließbar ist und an dessen anderen Ast die weiteren nachfolgend erläuterten Bestandteile der Harnsammelvorrichtung angeschlossen werden.

Die Harnsammelvorrichtung umfasst darüber hinaus einen in nachfolgend noch näher geschilderter Weise an das proximale Ende 10 des Blasenkatheters 1 angeschlossenen Verbindungsschlauch 40, der zu einem Sammelbehältnis 4 führt. Dieses schematisch dargestellte Sammelbehältnis 4 kann beispielsweise ein Urinbeutel oder auch ein Urinmesssystem sein.

Wie in näheren Einzelheiten aus der vergrößerten Darstellung gemäß Figur 2 ersichtlich, erfolgt der Anschluss des Verbindungsschlauches 40 gemäß einer ersten Ausführungsform in der Weise, dass das dem Sammelbehältnis 4 abgewandte freie Ende 41 des Verbindungsschlauches 40 in einen Adapter 2 in eine entsprechende Aufnahmebohrung 22a eingesetzt ist. Der Adapter ist vorzugsweise als Spritzgussteil aus einem thermoplastischen Kunststoff gefertigt.

Auf der der Aufnahmebohrung 22a abgewandten Seite weist der Adapter 2 einen zylindrischen Einsteckvorsprung 21 a auf und ist mit diesem in einen handelsüblichen Stufenkonnektor 3 im Bereich einer zylindrischen Einsteckbohrung 31 einsteckbar. Sodann wird der Stufenkonnektor 3 mit seinem abgestuften Einsteckbereich 30 am gegenüberliegenden Ende in an sich bekannter Weise in das proximale Ende 10 des Blasenkatheters 1 eingesteckt.

Die solchermaßen komplettierte Harnsammelvorrichtung bildet vom distalen Ende 11 bis zum Sammelbehältnis 4 ein durchgehendes Lumen L aus, so dass eine entsprechende Ableitung von Harn aus der Blase in das Sammelbehältnis 4 erfolgen kann.

Besonderes Merkmal des vorangehend erläuterten Adapters 2 ist es, dass dieser darüber hinaus über einen in das Lumen L einmündenden Messkanal 20 verfügt, an welchen eine nicht dargestellte Druckmessleitung anbringbar ist, um z.B. eine intra-abdominale Messung des Druckes des Patienten durchzuführen.

Wird eine solche Druckmessung nicht benötigt, so kann das freie Ende 41 des Verbindungsschlauches 40 in die Einsteckbohrung 31 des Stufenkonnektors 3 eingesetzt und der Adapter 2 entfernt werden.

In gleicher Weise ist es möglich, bei einer bereits gelegten Harnsammelvorrichtung mit einem über den Stufenkonnektor 3 angeschlossenen Verbindungsschlauch 40 zum Sammelbehältnis 4 bei Bedarf die Verbindung zwischen Verbindungsschlauch 40 und Stufenkonnektor 3 zu unterbrechen und den Adapter 2 in der in der Figur 2 dargestellten Konfiguration dazwischenzuschalten, so dass das Lumen verlängert wird, gleichzeitig aber die Druckmessung über den Messkanal 20 des Adapter 2 ermöglicht wird. Ein Teilaustausch der Harnsammelvorrichtung im Falle einer gewünschten Druckmessung ist somit nicht notwendig und es kann weiterhin auf die Standardkonfiguration von Stufenkonnektoren 3 zurückgegriffen werden.

In einer alternativen Ausgestaltung gemäß Figur 3 erfolgt der Anschluss des Verbindungsschlauches 40 an das proximale Ende 10 des Blasenkatheters 1 dadurch, dass der Verbindungsschlauch 40 zunächst, wie in der Standardkonfiguration, mit seinem freien Ende 41 in die Einsteckbohrung 31 eines Stufenkonnektors 3 eingesetzt wird. Sodann wird ein Adapter 2 verwendet, der auf seiner dem Stufenkonnektor 3 zugewandten Seite eine in das Innere des Adapters 2 konisch verengende Einstecköffnung 22 aufweist, so dass der Stufenkonnektor 3 mit seinem abgestuften Einsteckbereich 30 in diese sich konisch verengende Einstecköffnung 22 einsteckbar ist. Der Adapter 2 ist vorzugsweise aus einem Elastomer gefertigt, wobei durch geeignete Wandstärken- und/oder Materialauswahl die Einstecköffnung 22 elastisch ausgebildet ist und der Stufenkonnektor 3 darin festklemmend befestigbar ist.

Auf seiner dem Stufenkonnektor 3 abgewandten Seite ist der Adapter 2 in einer mit dem Einsteckbereich 30 des Stufenkonnektors 3 übereinstimmend abgestuften Außenprofilierung 21 versehen, so dass über dieses abgestufte Außenprofil 21 eine Verbindung mit dem proximalen Ende 10 des Blasenkatheters 1 hergestellt werden kann. Hier ist durch geeignete Wandstärken- und/oder Materialauswahl der Adapter 2 rigide ausgeführt, so dass er die Funktion eines Stufenadapters ausführen kann.

Im Rahmen der Erfindung kann der Adapter z.B. im Spritzgussverfahren gefertigt werden, wobei die Elastizität bzw. Rigidität z.B. durch Einsatz eines 2-Komponenten-Spritzgussverfahrens in weiteren Grenzen variierbar ist.

Auch der in der Figur 3 dargestellte Adapter 2 verfügt über einen in das durchgehende Lumen einmündenden Messkanal 20 für den Anschluss einer intraabdominalen Druckmessleitung, so dass auch in diesem Falle eine IAP-Messung ermöglicht ist, ohne dass die Verwendung von speziell ausgebildeten Harnsammelvorrichtungen erforderlich wäre.

Nach Entfernung des Adapters 2 kann überdies die verbleibende Harnsammelvorrichtung wieder zusammengefügt und in konventioneller Weise genutzt werden.

Die in den vorangehenden Ausführungsbeispielen dargestellten Adapter 2 können beispielsweise als Spritzgussteile aus einem geeigneten thermoplastischen Kunststoff oder einem Elastomer in großindustriellem Maße wirtschaftlich hergestellt werden, z.B. aus Silikon. Silikon hat überdies die Eigenschaft, die Entstehung von Harnsäurekristallen zu hemmen, so dass der Adapter 2 auch über längere Zeiträume im System verbleiben kann, so dass Langzeit- und/oder Mehrfachmessungen möglich sind.

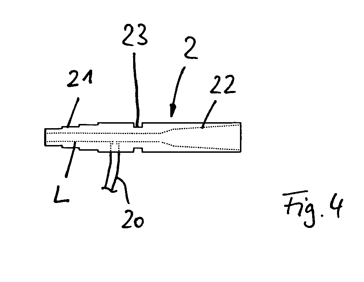

Aus der Ausgestaltung gemäß Figur 4 ist eine Abwandlung des Adapters 3 gemäß Figur 3 ersichtlich. Der Adapter weist in seinem mittleren Bereich vom abgestuften Außenprofil 21 her gesehen hinter der Messleitung 20 eine Umfangsnut 23 auf, auf der eine nicht dargestellte Schiebeklemme an eindeutig definierter Position zum Verschließen des Lumens L angebracht, beispielsweise vormontiert werden kann. Ausgehend vom Messkanal 20 liegt die Umfangsnut 23 somit auf der dem proximalen Ende 10 des Blasenkatheters 1 abgewandten Seite des Adapters, so dass der Fluss aus dem Blasenkatheter 1 in das Sammelbehältnis 4 unterbrochen werden kann, gleichwohl jedoch der verbleibende Teilabschnitt des Lumens L, die Messleitung 20 sowie der Blasenkatheter 1 noch miteinander verbunden ist. Somit ist während einer Druckmessung die Befüllung der Patientenblase mit einem vorgebbaren Volumen an Flüssigkeit und die anschließende Druckmessung mit höchster Genauigkeit möglich.

Selbstverständlich ist eine Umfangsnut gemäß Figur 4 auch auf das Ausführungsbeispiel gemäß Figur 2 übertragbar.

## Patentansprüche

1. Adapter für eine Harnsammelvorrichtung, die einen Blasenkatheter (1) mit proximalem und distalem Ende (10, 11) sowie ein Sammelbehältnis (4) mit Verbindungsschlauch (40) umfasst, wobei der Verbindungsschlauch (40) über einen Stufenkonnektor (3) an das proximale Ende (10) des Blasenkatheters (1) unter Ausbildung eines vom distalen Ende (11) bis zum Sammelbehältnis (4) durchgehenden Lumens (L) anschließbar ist, **dadurch gekennzeichnet, dass** der Adapter (2) unter Verlängerung des Lumens (L) zwischen dem proximalen Ende (10) des Blasenkatheters (1) und dem Stufenkonnektor (3) oder dem Stufenkonnektor (3) und dem Verbindungsschlauch (40) einsetzbar ist und einen in das Lumen (L) einmündenden Messkanal (20) für eine Druckmessung aufweist.

2. Adapter nach Anspruch 1, **dadurch gekennzeichnet, dass** er für den Einsatz zwischen Blasenkatheter (1) und Stufenkonnektor (3) auf seiner dem proximalen Ende (10) des Blasenkatheters (1) zugewandten Seite mit einem abgestuften Außenprofil (21) zum Aufstecken des proximalen Endes (10) des Blasenkatheters und auf seiner dem Stufenkonnektor (3) zugewandten Seite mit einer sich konisch verengenden Einstecköffnung (22) zum Einstecken des Stufenkonnektors (3) ausgebildet ist.

3. Adapter nach Anspruch 1, **dadurch gekennzeichnet, dass** er für den Einsatz zwischen Stufenkonnektor (3) und Verbindungsschlauch (40) auf seiner dem Stufenkonnektor zugewandten Seite mit einem zylindrischen Einsteckvorsprung (21a) zum Einstecken in den Stufenkonnektor (3) und auf seiner dem Verbindungsschlauch (40) zugewandten Seite mit einer Aufnahmebohrung (22a) zum Einsetzen des freien Endes (41) des Verbindungsschlauches (40) ausgebildet ist.

4. Adapter nach einem der Ansprüche 1 bis 3, **dadurch gekennzeichnet, dass** er ausgehend vom Messkanal (20) auf seiner dem proximalen Ende (10) des Blasenkatheters (1) abgewandten Seite mit einer Umfangsnut (23) ausgebildet ist und eine Abklemmvorrichtung in der Umfangsnut (23) führbar ist.

5. Adapter nach einem der Ansprüche 1 bis 4, **dadurch gekennzeichnet, dass** er als Spritzgussteil aus einem thermoplastischen Kunststoff oder einem Elastomer oder einer Kombination derselben gefertigt ist.

6. Adapter nach Anspruch 5, **dadurch gekennzeichnet, dass** er aus einem Silikon gefertigt ist.

7. Harnsammelvorrichtung mit einem Adapter (2) gemäß einem der vorangehenden Ansprüche.
